(19) **Europäisches Patentamt / European Patent Office / Office européen des brevets**

(11) **EP 1 520 034 B1**

(12) # EUROPEAN PATENT SPECIFICATION

(45) Date of publication and mention
of the grant of the patent:
**23.02.2011 Bulletin 2011/08**

(21) Application number: **03735209.3**

(22) Date of filing: **26.06.2003**

(51) Int Cl.:
*C12Q 1/22* (2006.01)     *A23L 3/00* (2006.01)

(86) International application number:
**PCT/BE2003/000114**

(87) International publication number:
**WO 2004/003226 (08.01.2004 Gazette 2004/02)**

(54) **ENZYME-BASED MONITORING DEVICE FOR THE THERMAL PROCESSING OF OBJECTS**

ENZYM BASIERTE ÜBERWACHUNGSVORRICHTUNG FÜR DIE HITZEBEHANDLUNG VON OBJEKTEN

DISPOSITIF DE CONTROLE ENZYMATIQUE POUR LE TRAITEMENT THERMIQUE D'OBJETS

(84) Designated Contracting States:
**DE ES FR GB**

(30) Priority: **26.06.2002 GB 0214749**

(43) Date of publication of application:
**06.04.2005 Bulletin 2005/14**

(73) Proprietors:
• **K.U. LEUVEN RESEARCH & DEVELOPMENT VZW**
  **B-3000 Leuven (BE)**
• **Centre Technique de la Conservation des Produits**
  **Agricoles**
  **75682 Paris Cédex 14 (FR)**

(72) Inventors:
• **GUIAVARCH, Yann**
  **F-54000 Nancy (FR)**
• **VAN LOEY, Ann**
  **B-3001 Heverlee (BE)**
• **HENDRICKX, Marc**
  **B-3001 Heverlee (BE)**

(74) Representative: **Bird, Ariane et al**
**Bird Goën & Co**
**Klein Dalenstraat 42A**
**3020 Winksele (BE)**

(56) References cited:
EP-A- 1 138 777     US-A- 5 486 459
US-A- 5 739 004     US-B1- 6 355 448

• HAENTJENS T H ET AL: "The use of alpha-amylase at reduced water content to develop time temperature integrators for sterilization processes." LEBENSMITTEL-WISSENSCHAFT UND -TECHNOLOGIE 31 (5) 467-472 1998 CORRESPONDENCE (REPRINT) ADDRESS, M. E. HENDRICKX, LAB. OF FOOD TECH., DEP. OF FOOD & MICROBIAL TECH., KATHOLIEKE UNIV. LEUVEN, B-3001, HEVERLEE, BELGIUM, XP002260356
• TUCKER G S ET AL: "Application of a biochemical time-temperature integrator to estimate pasteurisation values in continuous food processes." INNOVATIVE FOOD SCIENCE AND EMERGING TECHNOLOGIES 3 (2) 165-174 2002 PROCESS & DEV. DEP., CAMPDEN & CHORLEYWOOD FOOD RES. ASS., CHIPPING CAMPDEN GL55 6LD, UK, XP001156113
• LOEY A VAN ET AL: "Quantitative evaluation of thermal processes using time-temperature integrators." TRENDS IN FOOD SCIENCE & TECHNOLOGY 1996 CORRESPONDENCE (REPRINT) ADDRESS, M. HENDRICKX, DEP. OF FOOD & MICROBIAL TECH., LAB. OF FOOD TECH., FAC. OF AGRIC. & APPLIED BIOL. SCI., KATHOLIEKE UNI, vol. 7, no. 1, pages 16-26, XP002260357
• CORDT S DE ET AL: "DSC and protein-based time-temperature integrators: case study of.Na-amylase stabilized by polyols and/or sugar." BIOTECHNOLOGY AND BIOENGINEERING 1994 CORRESPONDENCE (REPRINT) ADDRESS, M. HENDRICKX, FOOD PRESERVATION UNIT, CENT. OF FOOD SCI. & TECH., FAC. OF AGRIC. & APPLIED BIOL. SCI., KATHOLIEKE UNIV., vol. 44, no. 7, pages 859-865, XP001156109

- DE CORDT S F ET AL: "CONVENIENCE OF IMMOBILIZED BACILLUS LICHENIFORMIS -AMYLASE AS TIME-TEMPERATURE-INTEGRATOR (TTI)" JOURNAL OF CHEMICAL TECHNOLOGY AND BIOTECHNOLOGY. (INTERNATIONAL JOURNAL OF BIOTECHNICAL AND CHEMICAL PROCESSES), ELSEVIER APPLIED SCIENCE PUBLISHERS. BARKING, GB, vol. 59, no. 2, 1 February 1994 (1994-02-01), pages 193-199, XP000425515 ISSN: 0268-2575
- HENDRICKX M ET AL: "Validation of a time-temperature-integrator for thermal processing of foods under pasteurization conditions." INTERNATIONAL JOURNAL OF FOOD SCIENCE & TECHNOLOGY 1992 LAB. OF FOOD TECH., KATHOLICKE UNIV. LEUVEN, 3001 HEVERLEE, BELGIUM, vol. 27, no. 1, pages 21-31, XP009020571
- LY NGUYEN B ET AL: "Purification, characterization, thermal, and high-pressure inactivation of pectin methylesterase from bananas (cv. Cavendish)." BIOTECHNOLOGY AND BIOENGINEERING 78 (6) 683-691 2002 CORRESPONDENCE (REPRINT) ADDRESS, I. M. HENDRICKX, LAB. OF FOOD TECH., DEP. OF FOOD & MICROBIAL TECH., FAC. OF AGRIC. & APPLIED BIOL. SCI., KATHOLIEKE UNIV. OF LEUVEN, B-3001 LEUVEN, BELGIUM. TEL. , 20 June 2002 (2002-06-20), XP002260359
- MAESMANS G ET AL: "Evaluation of process value distribution with time temperature integrators." FOOD RESEARCH INTERNATIONAL 1994 CORRESPONDENCE (REPRINT) ADDRESS, M. HENDRICKX, FAC. OF AGRIC. & APPLIED BIOL. SCI., CENT. FOR FOOD SCI. & TECH., KATHOLIEKE UNIV. TE LEUVEN, B-3001, LEUVEN, B, vol. 27, no. 5, pages 413-423, XP009020504

**Description**

The present invention pertains to the field of thermal processing of objects, such as food, under sterilization or pasteurization conditions and more particularly relates to an enzyme-based monitoring device for monitoring fodder, human or animal food or other object thermal processing especially under pasteurization or sterilization conditions. The invention also relates to the use of a dehydrated enzyme-filler mix as a bio-integrator for monitoring the thermal processing of objects, e.g. in the form of a pasteurization or sterilization process step. In addition, this invention relates to a method of monitoring the thermal impact of thermal processing on an object.

BACKGROUND OF THE INVENTION

Nowadays, an important part of the human or livestock or animal food supply chain consists of foods preserved through a thermal treatment such as blanching, pasteurisation or sterilisation. In this context it is important for food manufacturers to measure the impact of thermal processes in term of safety and quality in the context of process design, optimization, evaluation and control.

The aim of food thermal processing is to preserve food against spoilage which could render the food unfit for consumption, by inactivation of pathogenic and/or spoilage microorganisms and of enzymes with deteriorative action. Unfortunately, along with these desired effects, thermal processing also affects both the nutritional and sensorial quality of the product. Food technology therefore tries to minimize quality losses and at the same time provide an adequate process to achieve the desired degree of lethality. This optimization is possible because of the more pronounced temperature dependence of microbial (spores) inactivation as compared to the rate of quality destruction, sensorial as well as nutritional. Hence, relevant knowledge of the kinetic data for heat induced inactivation of microorganisms and quality evolution is essential to optimize thermal processes. In addition, for preservation purposes, the quantitative measurement of the impact of a thermal process in terms of safety and quality is important for process design, evaluation and optimization to obtain a safe food product with maximal quality retention.

The impact of a heat treatment on a food product safety or quality attribute depends on the rates of the heat-induced reactions that affect this attribute, and on the time interval during which these reactions occur.

The status of a food product, expressed in terms of its safety or quality, is determined by the effect of all reactions occurring in the product, integrated over the full history of the product until the moment of consumption. This concept can be applied to an entire food production chain (including preparation and packaging, processing, distribution and storage) or it is applicable to a single unit operation. The rates at which desired and undesired reactions (related to food safety and quality) take place are function of both intrinsic (i.e. food specific) properties and extrinsic (i.e. process specific).

The processing of fodder for livestock is also a potential source of risk both for the livestock themselves (e.g. so-called " mad cow disease ") and possibly even to human consuming meat from infected animals. The European Council Directive 90/667/EEC of 27 November 1990, Annex II, chapter II, section G, requires that high risk animal waste must be processed to a core temperature of 133°C for 20 minutes at a pressure of 3 bar. However, due to dead zones on rapid transport of some particles, the required residence may not be achieved.

Because in food thermal processing, temperature is the main extrinsic factor for guaranteeing safety and quality during the production and storage of food products, the present invention mainly deals with systems for which temperature is the only rate determining extrinsic factor.

Two models are commonly used to quantify the influence of temperature on the inactivation rate of safety as well as of quality aspects: the Arrhenius model (1889) commonly used in the field of chemical reaction kinetics and the Thermal Death Time model (1921), especially for first order kinetics and commonly used in thermo-bacteriology and in the field of thermal processing. These two models are well known in the art and need not be further described here.

The impact of a thermal treatment on a food attribute is indeed usually quantified using the concept of an " equivalent time at a reference temperature ", referred to as the processing value F (usually expressed in minutes). This concept translates the time-temperature variable profile into an equivalent time chosen at a constant reference temperature that will affect the quality attribute in the same way as the variable profile. In other words, F represents the equivalent time at a chosen constant reference temperature $T_{ref}$ that would result in exactly the same impact on the specific quality attribute as the actual time-temperature variable profile to which the food (i.e. the attribute of interest) was subjected. Mathematically, F can be written in terms of the temperature history of the product or alternatively in terms of a response status before and after processing. The impact of a heat treatment on a product safety or quality attribute depends on the rates of the heat-induced reactions affecting this attribute and on the reaction time interval during which these reactions rates occur.

In practice, isothermal heating profiles almost never occur because heating equipments require time to reach the process holding temperature and require time for cooling and because of the heat transfer inside the product. Hence, reaction rates are varying as a function of process time. The process-value is defined as the integral over time of the rate at each encountered temperature relative to the rate at a chosen reference temperature.

Hence, based on the temperature history of the product aspect, either recorded or simulated, combined with knowledge on the kinetics of the monitored aspect, the thermal process impact on that attribute can be calculated as mentioned above, this approach is commonly referred to as the physical mathematical method.

Furthermore, the impact of a thermal process on a specific parameter can also be determined relying solely on the initial and final status of the parameter of interest and on its kinetics. Based on the response status of an attribute before and after thermal treatment, combined with its kinetics, the process impact can be calculated depending on the order of the heat induced reaction occurring to this attribute. In case the level of the actual quality attribute of interest is measured before and after processing, this approach is referred to as the *in-situ* method.

As a summary, the *in-situ* method and the physical-mathematical approach are two common evaluation techniques in food thermal treatment control. In the *in-situ* method the level of a quality or safety attribute (i.e. thiamine content) is measured in the food itself before and after heat treatment, while the physical-mathematical method is based on the knowledge of the time-temperature history of the product, combined with the knowledge of heat resistance parameters of the food quality attribute under study.

In the *in situ* method, changes in the actual quality or safety attribute under study are monitored before and after processing to provide direct and accurate information on the status of the attribute of interest. The processing value can be calculated depending on the order of the heat-induced reaction occurring to the target attribute. The main advantage of the *in-situ* method is that the impact of the process on the parameter of interest is directly and accurately known. However, in practice, the analysis of the parameter under investigation (i.e. measurement of microbial counts, texture, vitamin content, etc.), can be laborious, time-consuming and/or expensive, and in some cases even impossible because of the detection limit of the analytical techniques at hand/or sampling requirements (e.g. for the safety of sterilized low-acid canned foods, public health policies impose that the probability of a non sterile unit should not exceed $10^{-9}$), which cannot be monitored.

In the physical-mathematical method, the calculation of the process value imposed on the food either requires the use of temperature sensors to record the time-temperature history at the critical point of the product or requires the use of theoretical solutions of heat transfer models or of semi-empirical methods (e.g. the method of Ball and Olson commonly used in industry) that use the knowledge of process parameters and heat penetration parameters of the food product itself combined with knowledge on the kinetics of the safety/quality attribute under study.

With regard to semi-analytical methods such as the method of Ball and Olson, several problems can arise:

- direct registration of the time-temperature profile of the product may not be possible under some processing conditions, e.g. cable thermal probes cannot be used in continuous thermal processing and wireless thermal sensors disturb too much the heat transfer in the product;
- the critical point of a product, except for real conductive products, is almost always moving during the heating process (whether they are discontinuous or continuous, a number of heat treatments submit products to axial or end over end rotation in order to take advantage of their convective characteristics to increase the heating rate).

Increasing safety and quality requirements, energy saving policies and consumers aspects, have resulted in the optimization of classical thermal technologies and the development and application of new (thermal as well as non thermal) technologies such as continuous processing in rotary retorts, volumetric heating before aseptic processing, use of high electric field pulses , high pressure processing, infrared technology, etc. Confronted with these new technologies, the *in-situ* and physical-mathematical methods show serious limitations. This is why considerable efforts have been put into the development of product history integrators such as Time Temperature Integrators (hereinafter referred as TTI) for thermal processing. TTI allow to fastly, easily and accurately determine post *factum* the impact of a certain thermal treatment on a product attribute without knowledge of the time-temperature history of the product.

Until now, mainly microbiological TTI are used for monitoring thermal food processing from a safety point of view. However, the inherent disadvantages associated with microbiological detection methods have encouraged the investigation of alternatives. The advantages and drawbacks of several kinds of TTI have been reviewed by Hendrickx et al. in Critical reviews in Food Science and Nutrition (1995) 35(3):231-262 and by Van Loey et al. in Trends in Food Science & Technology (1996) 7(1):16-26.

Several enzymic systems have been developed to evaluate the impact of pasteurization and sterilization processes on, among others, microbiological target attributes such as *Clostridium botulinum* spores. Although these α-amylase based TTI systems are valuable research tools, the need of an industrially applicable enzymic system that is accurate, inexpensive and convenient to use for the monitoring of large scale sterilization processes still exists.

A TTI can be defined as a small wireless measuring device that shows a time-temperature dependent, easily, accurately and precisely measurable irreversible change that mimics the change of a target attribute undergoing the same variable temperature exposure. Its main advantage is the ability to quantify the integrated time-temperature impact on a target attribute without the need for information on the actual temperature history of the product. Ideally, a TTI should meet the following criteria:

1) be inexpensive, quickly and easily prepared, easy to recover and give an accurate and user-friendly read-out,

2) the TTI should be incorporated in the food without disturbing heat transfer within the food; the presence of the TTI must not change the time temperature profile of the food, and the TTI should be exposed to the same time-temperature profile as the target attribute under investigation,

3) the TTI should quantify the impact of the process on the target attribute under study, i.e. has to meet kinetic requirements. The temperature dependency of the rate constants of TTI and target attribute should be described by the same model (e.g. the Arrhenius model, or the Thermal Death Time model). As a kinetic requirement it is also important that the TTI possess an acceptable heat inactivation rate value at the desired processing temperature. If said value is too high, the TTI can be almost totally inactivated so that the TTI response may be below the detection limit of the reading method. At the opposite, a too low value may lead to a change in TTI response which is too limited for an accurate detection of changes.

TTI can be classified in terms of working principle, type of response, origin, application in the food material and location in the food as shown in Hendrickx et al.(cited *supra*). Depending on the working principles, TTI can be subdivided into biological (microbiological and enzymic), chemical and physical systems. Relating to their origin, extrinsic and intrinsic TTI can be distinguished.

With regard to the application of the TTI in the food product, three approaches (dispersed, permeable or isolated) can be distinguished:

-    in dispersed systems, the TTI (extrinsic or intrinsic) is homogeneously distributed throughout the food, allowing evaluation of the volume-average impact of a process; besides dispersion of an extrinsic TTI in the food, extrinsic TTI may be permeable (permitting some diffusion of food components into the TTI) or isolated.
-    in dispersed and permeable TTI, temperature is no longer the only factor that can influence the TTI response. Intrinsic properties of the food such as salinity, pH etc...can also have an influence on the TTI kinetics. These intrinsic properties should be taken into account when developing a dispersed or permeable TTI. Hence, it remains possible to calculate a process value because the TTI and the target attribute show the same inactivation kinetics under the same environmental conditions of the sterilization process.

Encapsulated TTI have also been proposed to avoid the influence of the food environment on the kinetic behavior of the TTI. In this case, the TTI is completely isolated from the food environment by embedding it in an inert carrier material such as glass, plastic or metal. Hence, temperature becomes the only influencing factor. It is necessary to choose a TTI carrier with a thermal resistivity as small as possible in order to ascertain that the observed TTI response is due to the food product heating and not limited by the heating of the carrier material. For instance, use of small hermetically sealed or screwed highly conductive metal carrier systems guarantees the elimination of any influence of the environment other than temperature and allows its incorporation into a real food product. Some of the required properties that any TTI-carrier system should meet include:

-    a thermal diffusivity as close as possible to that of food,
-    in the case of particulate food heated in a rotary process (e.g. end over end rotation) or a continuous process (e.g. aseptic processing), the TTI carrier should possess (once filled with TTI) the same density and, if possible, a similar shape as the critical particle in order to mimic as close as possible the movement of the particle during the process. For each liquid or particulate food, a prior study of thermal conductivity, shape, density needs to be performed in order to determine the critical particle that has to be mimicked by the carrier,
-    the TTI carrier should possess an acceptable mechanical resistance in order to keep its shape when submitted to mechanical stress (e.g. particulate food pumped during volumetric heating processes or surface scraped heat exchanger), and
-    the carrier material should be adapted to the heating process used (e.g. avoid the use of metallic carrier with ohmic or microwave heating).

*Bacillus sp.* are the most commonly used for the development of microbial TTI in the food and pharmaceutical industries. One can distinguish two kinds of microbial TTI:

-    count reduction systems which allow to calculate a process value, and
-    survivor/kill systems that only allow to identify whether the thermal process was sufficient (no growth) or not (growth) to kill a given amount of microorganisms present in the food.

The major disadvantage of any microbial monitoring system is the time required to perform the assay. The long incubation time (from 24 hours up to several days) and read-out of the system does not allow for rapid intervention upon any kind of (systematic) failure or process deviation. Quantitative microbiology has to be performed by skilled manpower and the analytical precision of currently available techniques is rather low. Heat resistance determination of spores requires thorough calibration. This step to determine the killing power of a given heat treatment is difficult to achieve. The inherent limitations of microbiological detection methods in determining the efficacy of thermal processing, together with the time and expense associated with these methods, have prompted the investigation of alternatives.

The potential of protein-based systems, in particular enzyme-based systems, is receiving considerable interest. The relative easiness of read-out and handling of enzymic systems offers significant advantage over microbial TTI. Moreover, the range of heat denaturation kinetics of proteins includes the typical values for both safety and quality aspects. In general, enzyme inactivation is characterized by a z value in the range from 6.5°C to 55°C, whereas the z-value of the thermal inactivation of vegetative cells and spores is 4.5 to 12°C and for quality aspects (e.g. color, texture, flavor, vitamins) in the range from 25 to 45°C. These characteristics are of interest with regard to safety considerations because the design of sterilization processes for low acid canned food are directed to the destruction of spores of proteolytic strains of *Clostridium botulinum* with a z-value of 10°C. For the evaluation of safety of pasteurization processes several microorganisms with z-values ranging from 5 to 12°C have been advanced for use as reference organism because depending on the type of food, intended shelf life, different microorganisms might be the main cause of poisoning. In enzyme based monitoring systems, often the enzymic activity remaining after the heat treatment is assayed to determine the thermal impact, although other properties, such as the heat of enzyme deterioration, can be determined instead (e.g. using Differential Scanning Calorimetry, hereinafter referred to as DSC).

Amylases from different *Bacillus* species have been most frequently studied. The feasibility of TTI using *Bacillus licheniformis* α-amylases covalently immobilized on glass beads or *Bacillus subtilis* α-amylase (at above 10% moisture content) or *Bacillus amyloliquefaciens* α-amylases in the presence of polyols, including carbohydrates, has been evaluated. Although these α-amylase based TTI systems are valuable research tools, they show the following limitations:

- the range of process values that can be investigated (at most 14 minutes) is too low with regard to large process values observed in industry (about 60 minutes or more),
- they are difficult and time-consuming (about 3 weeks) to prepare,
- they require a significant amount of enzyme (about 10 mg per TTI or more),
- residual enthalpy of denaturation, requiring expensive DSC equipment, is used as the main response property,
- residual activity is hardly used as a response property because protein aggregation phenomena cause difficulties in solubilizing the enzyme before activity reading,
- they are very poorly stable during storage between preparation and use,
- they are very poorly stable during storage between heating and reading.

As a consequence, an industrially applicable, accurate, inexpensive and convenient enzymic system for industrial applications is still a need in the art.

U.S. Patent No. 5,486,459 discloses a sterilization indicator comprising enzymes immobilized on a cellulose disc and freeze dried. The disc is inserted into a vial and covered with mineral oil. A heat resistant sponge stopper is used to hold the mineral oil and disc in place in the vial.

## SUMMARY OF THE INVENTION

In order to overcome the limitations of the enzyme-based systems of the prior art, the present invention provides an efficient and standardized enzymic TTI system and method which allows to use a very small amount, preferably an amount not above 3 mg, of an enzyme as a component of an accurate, inexpensive and convenient time-temperature integrator, when showing first order inactivation, for monitoring thermal (in particular sterilization) processing impact on human or animal food, livestock fodder, medical tools and other objects. This procedure is suitably applicable to *Bacillus licheniformis* amylase and *Bacillus subtilis* α-amylase, although a number of other enzymes may be used as well.

The system and method of the invention makes use, in combination with this very small amount of an enzyme, of a first filler preferably in the form of non-porous beads (e.g made from glass) in order to avoid the aggregation phenomena mentioned herein-above. Several advantages derive from the invention. The main advantage is enzymes are efficiently diluted within the filler, for instance spread or adsorbed all over the surfaces of the beads, to avoid aggregation during heating that leads, after heating, to a protein network impossible to solubilize for subsequent activity measurement. Hence glass beads are used not only as a simple adsorption surface allowing to fix the enzyme, but also as a filler separating the enzyme molecules from each other.

Another important advantage is that a very small amount of enzyme, preferably an amount not above 3 mg, can be used to prepare the TTI of the invention and that this small amount can easily be solubilized for further residual activity determination.

DETAILED DESCRIPTION OF THE INVENTION

In a first aspect, this invention provides an enzyme-based monitoring device for monitoring the thermal impact of thermal processing on an object within a temperature range from 80°C to 160°C, said device comprising a container containing at least one enzyme and at least one barrier, wherein:

- said container is a hermetically sealed container, and
- said container encloses a solid dehydrated mix comprising said at least one enzyme and at least one first filler, wherein the water content of said dehydrated mix is below 0.6 by weight, hermetic sealing of the hermetically sealed container being obtained by means of said at least one barrier in order to prevent entry of moisture into said container, e.g. when the latter is placed within a high moisture content atmosphere during thermal processing of the object. Moisture vapor barrier materials are well known in the art and include, for instance, a variety of silicon-containing polymers.

In the device according to the invention, the water content of the said solid dehydrated mix is below 0.6% by weight, more preferably below 0.3% by weight, and still more preferably below 0.1% by weight of the said mix. Determination of the residual water content in the said solid dehydrated mix may be performed by any analytical method standard in the art, such as but not limited to gravimetry (loss on drying) or coulometric Karl Fisher methodology. Details of such methods may be found for instance in the U.S.A. Food and Drug Administration Guidelines (January 1990) for residual moisture in dried biological products.

Preferably in the device according to the invention, the at least one enzyme represents between 0.001 and 10% by weight, preferably between 0.02 and 5% by weight, of the solid dehydrated mix enclosed in the sealed container, whereas the at least one first filler represents between 90 and 99.999 % by weight, preferably between 95 and 99.98% by Weight, of the solid dehydrated mix.

Preferably the at least one first filler is a non-porous filler which may be either an inorganic filler, for instance selected from the group consisting of glass beads, metal beads and silica beads or an organic filler, for instance polymer beads.

The geometrical shape of the first filler is not critical to the present invention, although a regular shape may be an advantage. Preferably the at least one first filler consists of beads with an average size below about 0.3 mm.

In many instances, it may be advantageous when the device of the invention further comprises at least one second filler which may represent up to about 10%, preferably up to 5%, more preferably up to 3%, by weight of the solid dehydrated mix.

The at least one second filler may be a water-soluble filler, and may be either an organic filler, for instance selected from the group consisting of polyols and carbohydrates such as mannitol, glycerol, sorbitol, lactitol, sucrose, trehalose and polyvinyl-alcohol, or an inorganic filler, for instance selected from the group consisting of alkali and alkaline-earth metal salts, such as alkali and alkaline-earth metal halides, e.g. sodium chloride, calcium chloride or potassium chloride. Combinations of an organic filler and an inorganic filler may provide an advantageous second filler profile.

The present invention is widely applicable to a large range of enzymes from bacterial, vegetal, animal or fungal origin. The invention is applicable to enzymes which belong to any of the six classes of the International classification and nomenclature of enzymes, i.e. oxidoreductase, transferase, hydrolase, lyase, isomerase and ligase. In particular, a suitable enzyme is a bacterial α-amylase *or* a pectin methyl esterase. If needed, other suitable enzymes may be selected from the group consisting of α-glucosidase, glucoamylase, urease, dehydrogenase, phosphatase, kinase, pullulanase, glucosyltransferase and the like. Suitable enzymes for the purpose of this invention also include thermostable archaeal or bacterial enzymes which show protein stability at extreme conditions, sometimes referred as extremophiles.

According to an advantageous embodiment of the invention, the amount of the at least one enzyme in the device is below about 3 mg, preferably not above 2 mg, more preferably not above 1 mg.

Preferably, the sealed container of the device according to the invention is made from one or more moisture-impermeable materials selected from the group consisting of glass, silica, metals (e.g. stainless steel, aluminium or titanium) and polymers, especially non-hydrolyzable polymers and polymers withstanding hot moisture atmospheres without degradation. The sealed container may be made from one or more layers, especially a multi-layered material may be suitable depending on the choice of the main container material. The container of the device according to this invention is preferably sealed in such a way as to prevent that the moisture content of the solid dehydrated mix enclosed within the said container exceeds about 0.6% by weight, preferably 0.3% by weight, more preferably 0.1 % by weight of the said mix. Methods and materials for sealing the container of this invention will be detailed herein below.

Also disclosed is the use of a solid dehydrated mix comprising at least one filler and at least one enzyme as a bio-integrator for monitoring the thermal processing of an object within a temperature range from about 60°C to 160°C, alternatively 70°C to 140°C, conveniently from 80°C to about 130°C. The said object may be present under any physical form, but preferably in an at least partly solid form, more preferably is in a particulate form which may be dispersed within a liquid. For instance the said object may be food such as preserves, in particular soup, vegetables, meat, fish or pre-cooked dishes including any of the latter, optionally in combination with a sauce or other kinds of condiment, being made in a continuous or discontinuous autoclave reactor.

In another embodiment, the said object may be any object that, for hygienic purpose may need to be disinfected under sterilization conditions, such as a medical tool to be used in surgery procedures, or a medical device to be implanted into the body of a patient, such as a bone or cartilage implant. The said object may also be a pharmaceutical composition, preferably in the form of a liquid, syrup, cream or paste.

Within this use monitoring the thermal processing of an object is preferably based on measuring the residual enzymatic activity after said thermal processing. Thus, it may be in the form of a process step in a pasteurization process (at temperatures ranging from about 60°C to 100°C) or a sterilization process (at temperatures ranging from about 100°C to 160°C, e.g. 140°C).

In a second aspect, this invention provides a method for monitoring the thermal impact of thermal processing on an object by means of an enzyme-based monitoring device, said device comprising a container containing at least one enzyme and at least one barrier, said method comprising the steps of:

(a) placing said enzyme-based monitoring device in contact with said object or in the neighbourhood of said object;
(b) exposing said object and said enzyme-based monitoring device to thermal processing at a temperature within a range from 80°C to 160°C for sufficient time for degrading a substantial portion of said at least one enzymewithout breaking said at least one barrier of said container;
(c) removing said container from contact with said object or from the neighbourhood of said object after completion of step (b);

characterised in that :

- said container is a hermetically sealed container,
- said container encloses a solid dehydrated mix comprising said at least one enzyme and at least one first filler, wherein the water content of said dehydrated mix is below 0.6% by weight, hermetic sealing of the hermetically sealed container being obtained by means of said at least one barrier in order to prevent entry of moisture into said container during thermal processing of said object, and
- said method further comprises the steps of:

(d) opening said hermetically sealed container and obtaining a sample of the at least one enzyme from said hermetically sealed container;
(e) measuring the residual activity of said at least one enzyme in the obtained sample, and
(f) using the measured residual activity as a means to quantify the thermal impact of the thermal processing of step (b) on one or more given target attributes of said object

Ideally, in view of the quantification step (f), the activity of said at least one enzyme should also be determined before thermal processing.

Within this second aspect of the invention, terms such as " object ", " mix ", "container ", " sealed ", " first filler " and the like should be understood as being defined in a similar manner as in the first and second aspects of the invention. The device of the invention is not required, for an efficient and accurate monitoring, to be in direct contact with the object submitted to thermal treatment, although it can be placed at the surface of the said object or, especially in the case of said object being food, inside the said food. It is sufficient for the device of the invention to be placed at short distance of the object submitted to thermal treatment, in such a way that the thermal history of the dehydrated mix enclosed within the sealed container is sufficiently similar to the thermal history of the object. Determination of the suitable maximum distance, depending upon the thermal treatment conditions (temperature and time) and upon the exact nature of the object is well beyond the Knowledge of the person skilled in the art.

Also, the proportions of the enzyme and/or the first (organic or inorganic, preferably non-porous) filler in the dehydrated mix, as well as other optional parameters such as but not limited to the amount of the enzyme and/or the optional presence of one or more second fillers and the proportions thereof in the dehydrated mix, are as disclosed herein-above with respect to the first aspect of the invention.

In a preferred and most convenient embodiment of the invention, the method for monitoring the thermal impact of thermal processing on an object according to the invention is further characterized in that :

- in step (d), a sample of the at least one enzyme from the said sealed container is obtained in the form of an enzyme solution by solubilizing in or more solvents the fraction of said solid dehydrated mix comprising the said at least one enzyme, and

- in step (e) the said enzyme solution is put into contact with a substrate for the said at least one enzyme, resulting in a product, and monitoring the residual activity of the said at least one enzyme is effected by quantifying the rate of formation of the said product.

The skilled person knows which solvent or combination of solvents may be suitable for satisfactorily solubilizing the at least one enzyme from the sample taken from the sealed container, depending from the specific nature of the said enzyme and depending upon the second filler(s) optionally present in the dehydrated mix.

Within the method for monitoring the thermal impact of thermal processing on an object according to the invention, the given target attribute of the said object to be quantified in step (f) may be any kind of target attribute that is relevant to the object involved. For instance, it may advantageously be a microbiological property of the said object which helps in checking and controlling the sanitary condition of the said object before any further use. It may also, especially in the case of the object being human or animal food or fodder, be a chemical, physical or organoleptic property that is relevant to the taste, nutritional value or visual appearance of food or fodder such as but not limited to viscosity, colour, sugar content, fat content, vitamin content and the like.

Because of the numerous advantages explained herein above, the present invention is applicable to a wide range of industries, including the human or animal food industry, the seafood industry, the rendering industry as well as to health care and medicine, and to a wide range of industrial processes wherein heat or other thermal processing is involved in one or more manufacturing steps. In particular, it is useful in the context of a Hazard Analysis Critical Control Points (hereinafter referred as HACCP) system. It will be appreciated that in all these fields of application, the device and method of the invention will contribute to better product safety, decreased spoilage and less expensive manufacturing procedures.

Further features and advantages of the invention will now be explained in further details from a practical point of view. However it should be understood that the present invention is not limited to these more specific features.

The present Invention allows obtaining accurate and reproducible TTI with a z-value in the range from about 8 to 18°C, preferably from with a standard deviation not above 0.8°C, preferably not above 0.5°C, thus allowing the determination of a process value F (such as defined in the background of the invention) with an acceptable accuracy.

More specifically, the invention makes use of a mix comprising a strongly dehydrated enzyme adsorbed at the surface of non porous glass beads together with stabilizing substances (such as sucrose and mineral salts) as a second filler. This mix is packed in a hermetically sealed container, e.g. small capsules coated by a hermetic silicone layer. Important is the use of a first filler, e.g. glass beads of about 0.25 mm diameter. In order to achieve this solid dehydrated mix, the invention makes use of an extra-dehydration step, inside an oven at a temperature well above the room temperature, followed by an immediate sealing of the capsules and an entrapment inside a silicone moisture barrier material. From these three steps (extra-dehydration, immediate sealing, and entrapment in silicone) derive several advantages:

- a good control of the level of dehydration of the mix inside each capsule and subsequently achievement of a good level of accuracy of the TTI response (TTI show reproducible and stable constant z-value),
- a high level of thermal stability allowing to determine thermal impact at temperature higher than 100°C and up to about 160°C, e.g. 140°C, and
- an ability to store the TTI between its preparation and its use and between its use and its reading.

Moreover, the packed mix is easy to remove rapidly from DSC capsules coated with silicone.

The enzyme of this packed mix shows the following functional characteristics:

- after determination of its heat inactivation parameters (D-values or z-value), it allows to monitor process-values at high temperature exposure; for instance, when using *Bacillus licheniformis* α-amylase (hereinafter referred as BLA), it is possible to monitor process-values up to around 70 minutes within a temperature range from about 100 to 140°C,
- it is easy, by addition of a small amount (about 1 ml) of water to the dehydrated mix contained in one container and rapid mixing, to quickly obtain a total or substantial desorption of the at least one enzyme from the surface of the first filler (beads),
- it shows a great stability (several months) of its D-values and z-value, when the sealed container (e.g. coated with silicone as a sealing means) is stored at ambient temperature,
- it shows an acceptable stability (several days in the case of BLA) of its residual activity after thermal processing.

Going into still more detail, the present invention will now be explained by reference to the following example.

EXAMPLE 1 - monitoring device and method based on an α-amylase.

For standardization of the enzyme environment, the two following steps are preferably required: (1) standardization of the liquid environment of the enzymes and (2) standardization of the concentrations of these enzymes in the liquid environment. The standardised liquid environment may contain sucrose and sodium chloride as exemplary stabilizers.

Mixing the standardized enzyme solution with a non-porous filler (e.g. glass beads) mainly results in adsorption of the enzymes at the surface of the inert filler. In case glass beads are used as a filler, the required volume of standardized enzyme solution is the exact volume necessary to fill in the spaces between the glass beads.

An example is given below when using a *Bacillus licheniformis* α-amylase (BLA) and glass beads for the preparation of an enzyme-based TTI;

1) take a sample of *Bacillus licheniformis* α-amylase (BLA) solution,

2) determine the protein content (in mg/ml) of the sample using a Bicinchoninic Acid Protein determination kit BCA1 commercially available from Sigma. If unknown, determine also the iso-electric point and the molecular weight by using an isofocusing method and SDS-PAGE method respectively,

3) prepare a buffer corresponding to the iso-electric point of the enzyme: for BLA, prepare 1000 ml of a 0.005M bis-tris buffer, pH=6.9 at 25°C, by dissolving 1.046 g of a bls-tris base (MW = 209.2) in about 900 ml of distilled water at 25°C, then adding 2.56 g dehydrated calcium chloride, 15.24 g sodium chloride, 20 g sucrose and 1.27 g potassium chloride; then titrating to pH 6.9 with HCl 0.1N at 25°C, and finally making up volume to 1000 ml with distilled water,

4) on the basis of the protein content obtained in step (2), dilute a sample of the BLA solution with the buffer in order to reach a protein concentration of 1 mg/ml,

5) ultra-filtrate 9 ml of the solution obtained in step (4) by using a MACROSEP 3000, 10000 or 30000 kD centrifugal device (commercially available from Pall Life Sciences, USA). Centrifugation is performed in 2 steps: (1) at 6000 rpm during 120 minutes at 4°C for ultra-filtration, then (2) at 6000 rpm during 1 minute at 4°C for recovering the retention product.

6) complete the retention product with the buffer prepared in step (3) in order to obtain 5 folds the volume of the BLA solution taken initially and contained in the 9 ml ultra-filtered in step (5),

7) fill 1.5 ml Eppendorf cups with 1.35 g of glass beads of about 0.25 mm diameter and add 0.281 ml of the standardized enzyme solution obtained in step (6) (this volume allows to just fill in spaces between glass beads),

8) seal the tip of a glass capillary with a flame and use this capillary, once cooled, to mix correctly the beads and the solution obtained in step (6) to achieve a clear brown homogeneous color.

Then a strong level of dehydration of the mix is achieved in two steps:

- freeze drying allows to remove most of the water contained in the mix, then
- extra-dehydration of the mix by evaporation drying in an oven at a temperature well above room temperature up to constant weight (control with a balance showing an sensitivity of 0.01 mg).

For instance, the dehydration procedure applied to the mix achieved in step (8) may be as follows:

9) dehydrate by freeze drying the content of the Eppendorf cups (e.g. by use of a Christ (Switzerland) freeze drier) with the following freeze-drying parameters: safety pressure heating 10 mbar, main drying pressure 0.180 mbar, ice condenser at -82°C, temperature of the shelve during main drying at +4°C, freeze-drying time 20 hours; this step allows to remove the water between beads under non-denaturing conditions. While water is eliminated by sublimation, the enzyme, the ions from calcium chloride, potassium chloride and sodium chloride, and sucrose accumulate at the surface of the glass beads. At the end of freeze drying, these compounds coat the beads and act as a weak cement between them. After release of the vacuum, a three-dimensional network made of glass beads, enzyme, salts, sucrose and air is obtained;

10) recuperate the content of the Eppendorf cups, which consists of a solid block because proteins, sugars and salts, act as cement between the beads. With help of a stainless-steel spatula, a homogeneous powder is obtained. This powder (a "mix") is made of beads of which the surface is covered by a thin layer of enzymes and stabilizers (sucrose and metal chlorides),

11) efficiently mix the content of the Eppendorf cups together inside a plastic tube to achieve a homogeneous mix and place the latter inside clean Eppendort cups;

12) store the Eppendorf cups containing the homogeneous mix inside a dessicator containing $P_2O_5$;

13) equip pan covers with O-rings (Perkin-Elmer, kit 0319-0029); place them on a glass plate with the O-ring face visible; let the whole dry at 102°C during 15 minutes in an oven; then, remove the plate containing the covers from the oven and walt for cooling; once cooling is achieved, turn each cover (O-ring face not visible);

14) fill the pans of 60 μl Large Volume Capsules (Perkin-Elmer, kit 0319-0029) with 40 to 60 mg of the dehydrated mix (this step should be performed extremely rapidly, e.g. using a spatula;

15)further-dehydrate the freeze dried mix by placing the filled pans at 102°C during 1 hour inside an oven (note this occurs without any inactivation of the enzyme.

Then encapsulation of the dehydrated mix In a small and hermetically closed container is effected to avoid that the dehydrated mix takes up any atmospheric moisture. For instance, the encapsulation procedure applied to the mix dehydrated in step (15) is described below:

16)after 1 hour drying, remove the pans from the oven one by one and seal rapidly (i.e. within less than 10 seconds) the covers onto them according to Perkin-Elmer recommendations; close the oven door between each sealing;

17)let the capsules containing the mix cool down to ambient temperature and store the whole inside a dessicator containing $P_2O_5$;

18)entrap the capsules, containing the dried homogeneous mix, inside Sylgard 184 silicone elastomer (commercially available from Dow Corning Chemical, Belgium) according to the following procedure:

- dispose the capsules in rows on a glass plate in such a way that each row is separated from another row by a distance of 0.5 cm minimum (the cover of the capsules has to be in contact with the plate),
- prepare Sylgard 184 silicone following the recommendations of Dow Corning Chemical (10 part of Part A with 1 part of B (curing agent),
- pour the liquid silicone on the plate containing the capsules up to cover the capsules with 1 mm of silicone,
- let dry at 102°C inside an oven during 20 minutes,
- recuperate the plate with the capsules and the solidified silicon and let cool at ambient temperature,
- with a cutter, cut silicone around each capsule to obtain a squared piece of silicone (1 cm of side) containing the capsule,
- turn each piece of silicone containing a capsule and, with help of a small glass capillary, add a thin layer (0.5 mm) of liquid silicone on the face of the capsule non-protected by silicone,
- dry at 102°C in an oven during 15 minutes, and
- recuperate the pieces of silicone containing the capsules and remove the silicone corners with a cutter in order to obtain an octogonal shape (a small punch can also be used).

TTI heat inactivation kinetic parameters (D-values and z-value) under isothermal conditions may first be determined by making use of the classical two-steps regression method as follows. Under isothermal conditions, a first order reaction can be written as equation (1):

$$\ln \frac{X}{X_0} = -k \cdot t \qquad\qquad (1)$$

wherein:

- $X_0$ is the initial response, value (e.g. an initial enzyme activity $A_0$),
- X is the response value after heating treatment (e.g. residual enzymic activity A),
- T is the exposure time (expressed in minutes), and
- K is the inactivation rate constant (expressed in $min^{-1}$)

or, in the thermal death time terminology, as equation (2)

$$\log \frac{X}{X_0} = -\frac{1}{D} \cdot t \qquad\qquad (2)$$

wherein D is the decimal reduction time, i.e. the time for one log reduction of the response at a given temperature, (the D-value can be calculated from the slope of the linear regression line of log ($X/X_0$) versus time).

On the basis of Bigelow equation (3) below, the thermal sensitivity of the decimal reduction time, expressed by the z-value (°C), can be estimated by linear regression of log D-values versus corresponding temperatures:

$$D_{T_2} = D_{T_1} \cdot 10^{\left(\frac{T_1 - T_2}{z}\right)} \qquad\qquad (3)$$

According to the literature, when determining the slope of a first order reaction, the best accuracy is obtained by placing the experimental points at the two borders of the experimental domain. In our case, for each exposure temperature investigated, the experimental domain corresponded to the time interval between time 0 and the maximal exposure time corresponding to the detection limit of the reading method (in our case reading of the residual activity of the enzyme). On the basis of our experiments, it was demonstrated that the inactivation kinetics of BLA at low moisture content followed a first order kinetics. Hence, it is possible to use only 2 exposure times (close to the borders of the experimental domain) per investigated temperature (time 0 excluded) to determine the D-values at each exposure temperature. Accuracy was also increased by using 4 points per time-temperature combination. Isothermal heating of the sample was carried out by immersing simultaneously several TTI inside an oil bath (Grant, England) with silicone oil from Fluka (ref. 85415). Cooling was performed in ice-water at 0°C.

Tables 1 and 2 below show the D-values and z-values and their associated standard error of regression (determined by linear regression) respectively for a TTI prepared with a mix based on BLA and a TTI prepared with a mix based on *Bacillus subtilis* amylase (hereinafter referred as BSA).

Residual activity of the enzymes used to prepare the TTI is then used as a response property. For instance, when using a TTI based on BLA, the α-amylase activity is measured by means of a UV-visible spectrophotometer (commercially available from Pharmacia under the trade name LKB Biochrom 4060) at 30°C by using a kit Cat. No. TR 25421 (manufactured by Thermo Trace Ltd., Melbourne, Australia), i.e. based on the final release of p-nitrophenol, which absorbs maximally at 405 nm.

Activity is expressed in terms of the change In optical density per minute, calculated by linear regression from a plot of the absorption versus reaction time between 2 and 4 minutes. A temperature of 30°C was used to perform the activity assay. Because residual activity is used as response property, a rapid and efficient way to put the enzyme from the mix into solution is necessary. The following method was used:

1) dispose the same number of Eppendorf cups than capsules to be read on a rack, and number each of them with a marker,
2) put an empty 1.5 ml Eppendorf cup with safe lock on the plate of an accurate balance with a sensitivity of 0.0001 g,
3) open a capsule by double lateral compression within less than 10 seconds,
4) collect some mix from the pan of the capsule inside the Eppendorf cup, close the latter, and put it on the balance; the value displayed by the balance corresponds to the amount of mix and should preferably be between about 15 mg and 60 mg, then redo step (2) to (4) for each capsule,
5) solubilize the enzyme as follows:

- add 1 ml of distilled water into the Eppendorf cup containing an amount of mix from the capsule,
- close the Eppendorf cup,
- mix the Eppendorf cup radially with help of a vortex (at 2000 rpm during 30 seconds,
- mix the Eppendorf cup axially with hand during 30 seconds (end over end rotation),
- using a pipette, transfer 1 ml of Amylase Reagent and 0.020 ml of the solution contained inside the Eppendorf cup into a bowl; mix immediately by inversion and incubate at desired temperature during 2 minutes,
- determine the slope of the absorbance versus time curve between 2 and 4 minutes, and repeat step (5) for each capsule;

6) from each slope, deduce a residual activity A in Units/liter following the kit manufacturer recommendations. If the observed residual activity is higher than 2000 units/liter, dilute the enzymic solution from the Eppendorf cup in order to obtain a lower activity and repeat the measurement;
7) using the observed residual activity A and the initial activity $A_0$ corresponding to the same amount of a non-heated mix from a non-heated capsule and the $D_{121.1°C}$-value of the TTI, calculate the process value $F_{TTI}$ using equation standard in the art.

Since real thermal processing conditions involve non-steady state temperature conditions, it was necessary to evaluate the performance of the TTI system of the invention under variable temperature conditions.

Various non-isothermal profiles were generated by placing the TTI inside 2.5 cm diameter silicon spheres (Dow Corning Sylgard 184 Silicone Elastomer, Belgium). Thermocouples were prepared using 0.081 mm diameter copper-constantan wires (Omega, Belgium) and Ellab connectors and were connected to a CMC-92 data acquisition system (TR9216, Ellab, Denmark). An accuracy of 0.1°C was obtained with each thermocouple by comparison with a reference quartz temperature sensor (Testo, Belgium). Spheres were placed simultaneously in an oil-bath (Grant, England) at 123.2°C and time-temperature recording was started at the moment of immersion. Process values Ft-T corresponding to each sphere were calculated using a recording time-step of 15 seconds, a reference temperature of 121.1 °C and a z-value equal to the z-value of the TTI obtained under isothermal conditions. Cooling was performed by immersion in water at 0°C in order to obtain a process values in the range of 3 to 60 minutes. The process-values F achieved with the TTI were plotted versus the process-values achieved by the physical-mathematical method, in order to validate the ability of the enzymic system to suitably integrate time and temperature under dynamic conditions.

On the basis of the results obtained, an absolute percentage error in actual process value by the TTI is determined according to equation (4) below:

$$\left| \% \text{ error} \right| = \left| \frac{F_{t-T} - F_{TTI}}{F_{t-T}} \right| \times 100 \tag{4}$$

One goal of the present inversion is to put at the disposal of the food and fodder industry an efficient an inexpensive tool for monitoring thermal process impacts on a given target attribute of a given product. In case the target attribute under study would show a z-value different from the one of the TTI obtained following the above described TTI preparation, it is obvious that deviations between process-value deduced from the TTI response and the actual process-value concerning the target attribute under study may become more or less important depending on the actual temperature history inside the product during the thermal process. This temperature history will vary depending on the thermal characteristics of the product (heat penetration parameters) and on the characteristics of the process (holding time, holding temperature, come up time, come down time). Two different methods can then be used to calculate a correction factor to apply to the process-value derived from the response of the TTI:

- a first method, relating to a single component TTI showing a z value different from the one of the target attribute under interest, consists in integrating the time-temperature profile (measured or simulated with a finite difference heat transfer model on infinite cylinder) undergone by the product with the z-value of the target attribute under study and the z-value of the TTI at hand. The difference in corresponding process values $^{zTarget}F_{Tref}$ and $^{zTTI}F_{Tref}$ correspond to a correction factor to apply to any TTI responses for the product and the process under consideration;
- a second method is related to the use of a multi-component TTI based on a mix containing at least two different enzymes; the concept of multi-component TTI is well described in the literature.

Table 3 shows kinetic parameters achieved when using the TTI based on a highly dehydrated mix prepared with BLA, as well as the absolute percentage of error (at z values from 10 to 13.3°C) under isothermal conditions. Whatever the set of kinetic parameters used, this TTI allows the measurement of process-values in the range of 0 to 60 minutes.

As bio-sensors or bio-integrators, the TTI prepared according to the above described procedure may be sensitive to storage time and conditions either between preparation and use or between heating and reading of the response. We therefore also investigated the influence of storage time under atmospheric conditions at ambient temperature (15 to 25°C).

Table 4 shows the evolution of the TTI-response versus storage time at ambient temperature and under atmospheric conditions. The values correspond to a TTI based on BLA. Process-values derived from a TTI submitted simultaneously to the same heat treatments and read after certain storage times up to 6 days are listed. The initial enzymic activity used to perform calculations is the initial activity deduced from an average of 3 non heated TTI at day 0.

Stability (less than 8% decay) of the process value derived from the TTI of the invention can be observed up to 6 days after heat treatment. Due to the freeze-drying step and the drying step (102°C in an oven) Included in the preparation procedure of the TTI, the latter is totally dehydrated and was observed to show a good stability during several months. Heat inactivation parameters (D-values and z-values) were constant for several months.

Table 1

| | Heat inactivation kinetic parameters | |
|---|---|---|
| Temperature (°C) | D-values (minutes) | Z-value (°C) |
| 114.9 | 154 | 13.3 |
| 120.9 | 47.4 | |
| 124.7 | 26 | |
| 127.2 | 18.8 | |

Table 2

| | Heat inactivation kinetic parameters | |
|---|---|---|
| Temperature (°C) | D-values (minutes) | Z-value (°C) |
| 121.1 | 54 | 16.1 |
| 124.9 | 32.6 | |
| 128.1 | 19.7 | |

Table 3

| Regression's type | Kinetic parameters $D_{121.1°C}$ (minutes) $Z(°C)$ | $F_{TTI}/F_{t-T}$ ($Z_{TTI}$ = 13.3°C) absolute error % | $F_{TTI}/F_{t-T}$ (z=10°C) absolute error % |
|---|---|---|---|
| Linear | 45.8 minutes 13.3°C | 13.45 | 13.47 |

Table 4

| $F_{TTI}$- storage time | Heating treatments | | | $D_{121.1°C}$-value* (min) | z-value (°C) |
|---|---|---|---|---|---|
| | A: 60 min. at 115.8°C | B: 40 min. at 121.1°C | C: 15 min. at 124.9°C | | |
| $F_{TTI}$-Day 0 | 22.2 | 40.0 | 28.5 | 78.7 | 12.9 |
| $F_{TTI}$-Day 1 | 21.2 | 39.7 | 24.9 | 79.4 | 13.5 |
| $F_{TTI}$-Day 3 | 21.0 | 39.9 | 23.9 | 78.7 | 13.9 |
| $F_{TTI}$-Day 6 | 20.6 | 38.5 | 26.6 | 82.0 | 12.8 |

EXAMPLE 2 - monitoring device and method based on a pectin-methyl esterase.

When applying the above described TTI preparation procedure of example 1 to a purified cucumber pectin-methyl esterase, the inactivation curve obtained at 102°C allows to calculate a D-value, of 65.8 minutes at 102°C. Although only one temperature exposure was investigated (i.e. no z-value was determined), this result shows that (i) it is possible to strongly increase the thermal stability of a plant related enzyme such as cucumber pectin-methyl esterase up to temperatures higher than 100°C and (ii) hence, it makes it possible to use this preparation method in order to prepare a TTI useful for high temperature pasteurization processes (i.e. within a range from about 90 to 110°C).

**Claims**

1.   An enzyme-based monitoring device for monitoring the thermal impact of thermal processing on an object within a temperature range from 80°C to 160°C, said device comprising a container containing at least one enzyme and at least one barrier, wherein:

- said container is a hermetically sealed container, and
- said container encloses a solid dehydrated mix comprising said at least one enzyme and at least one first filler, wherein the water content of said dehydrated mix is below 0.6 by weight, hermetic sealing of the hermetically sealed container being obtained by means of said at least one barrier in order to prevent entry of moisture into said container.

2. An enzyme-based monitoring device according to claim 1, wherein said at least one enzyme represents between 0.001 and 10% by weight, of the solid dehydrated mix enclosed in said hermetically sealed container, and wherein said at least one first filler represents between 90 and 99.999 % by weight of the solid dehydrated mix enclosed in said hermetically sealed container.

3. An enzyme-based monitoring device according to claim 1 or claim 2, wherein said at least one first filler is a non-porous filler.

4. An enzyme-based monitoring device according to any of claims 1 to 3, wherein said at least one first filler is an inorganic filler or an organic filler.

5. An enzyme-based monitoring device according to any of claims 1 to 4, wherein said solid dehydrated mix further comprises at least one second filler which represents up to 10 % by weight of the solid dehydrated mix enclosed in said hermetically sealed container.

6. An enzyme-based monitoring device according to any of claims 1 to 5, wherein said at least one enzyme is from bacterial, vegetal, animal or fungal origin and wherein the amount of said at least one enzyme in said device is below about 3 mg.

7. An enzyme-based monitoring device according to any of claims 1 to 6, wherein said hermetically sealed container is made from one or more moisture-impermeable materials selected from the group consisting of glass, silica, metals and polymers.

8. A method of monitoring the thermal Impact of thermal processing on an object by means of an enzyme-based monitoring device, said device comprising a container containing at least one enzyme and at least one barrier, said method comprising the steps of:

(a) placing said enzyme-based monitoring device in contact with said object or in the neighbourhood of said object;
(b) exposing said object and said enzyme-based monitoring device to thermal processing at a temperature within a range from 80°C to 160°C for sufficient time for degrading a substantial portion of said at least one enzyme without breaking said at least one barrier of said container,
(c) removing said container from contact with said object or from the neighbourhood of said object after completion of step (b);

**characterised in that**:

- said container is a hermetically sealed container,
- said container encloses a solid dehydrated mix comprising said at least one enzyme and at least one first filler, wherein the water content of said dehydrated mix is below 0.6% by weight, hermetic sealing of the hermetically sealed container being obtained by means of said at least one barrier in order to prevent entry of moisture into said container during thermal processing of said object, and
- said method further comprises the steps of:

(d) opening said hermetically sealed container and obtaining a sample of the at least one enzyme from said hermetically sealed container,
(e) measuring the residual activity of said at least one enzyme in the obtained sample, and
(f) using the measured residual activity as a means to quantify the thermal impact of the thermal processing of step (b) on one or more given target attributes of said object.

9. A method of monitoring the thermal impact of thermal processing on an object according to claim 8, **characterized in that**:

- in step (d), a sample of said at least one enzyme enclosed in said hermetically sealed container is obtained in the form of an enzyme solution by solubilizing in or more solvents the fraction of said solid dehydrated mix comprising said at least one enzyme, and
- in step (e) said enzyme solution is put into contact with a substrate for said at least one enzyme, resulting in a product, and measuring the residual activity of said at least one enzyme is effected by quantifying the rate of formation of said product.

10. A method of monitoring the thermal impact of thermal processing on an object according to claim 9, wherein said given target attribute of said object being quantified in step (f) is a chemical, physical, organoleptic or microbiological property of said object.

11. A method of monitoring the thermal impact of thermal processing on an object according to any of claims 8 to 10, wherein said object is selected from the group consisting of human or animal food and medical tools or devices.

12. A method of monitoring the thermal impact of thermal processing on an object according to any of claims 8 to 10, wherein said object is a pharmaceutical composition in the form of a liquid, syrup, cream or paste.

13. A method of monitoring the thermal impact of thermal processing on an object according to any of claims 8 to 12, wherein said at least one enzyme represents between 0.001 and 10% by weight of said solid dehydrated mix.

**Patentansprüche**

1. Überwachungsvorrichtung auf Enzymbasis zum Überwachen der Wärmeauswirkung einer Wärmeverarbeitung auf ein Objekt innerhalb eines Temperaturbereichs von 80°C bis 160°C, wobei die Vorrichtung einen Behälter umfasst, der mindestens ein Enzym und mindestens eine Sperrschicht enthält, wobei:

- der Behälter ein hermetisch abgedichteter Behälter ist, und
- der Behälter eine feste, dehydrierte Mischung umschließt, die das mindestens eine Enzym und mindestens ein erstes Füllmittel umfasst, wobei der Wassergehalt der dehydrierten Mischung unter 0,6 Gew.% ist, wobei die hermetische Abdichtung des hermetisch abgedichteten Behälters mit Hilfe der mindestens einen Sperrschicht erhalten wird, um einen Eintritt von Feuchtigkeit in den Behälter zu verhindern.

2. Überwachungsvorrichtung auf Enzymbasis nach Anspruch 1, wobei das mindestens eine Enzym zwischen 0,001 und 10 Gew.% der festen dehydrierten Mischung darstellt, die in dem hermetisch abgedichteten Behälter eingeschlossen ist, und wobei das mindestens eine erste Füllmittel zwischen 90 und 99,999 Gew.% der festen dehydrierten Mischung darstellt, die in dem hermetisch abgedichteten Behälter eingeschlossen ist.

3. Überwachungsvorrichtung auf Enzymbasis nach Anspruch 1 oder 2, wobei das mindestens eine erste Füllmittel ein nicht poröses Füllmittel ist.

4. Überwachungsvorrichtung auf Enzymbasis nach einem der Ansprüche 1 bis 3, wobei das mindestens eine erste Füllmittel ein anorganisches Füllmittel oder ein organisches Füllmittel ist.

5. Überwachungsvorrichtung auf Enzymbasis nach einem der Ansprüche 1 bis 4, wobei die feste dehydrierte Mischung des Weiteren mindestens ein zweites Füllmittel umfasst, das bis zu 10 Gew.% der festen dehydrierten Mischung darstellt, die in dem hermetisch abgedichteten Behälter eingeschlossen ist.

6. Überwachungsvorrichtung auf Enzymbasis nach einem der Ansprüche 1 bis 5, wobei das mindestens eine Enzym bakteriellen, pflanzlichen, tierischen oder pilzlichen Ursprungs ist und wobei die Menge des mindestens einen Enzyms in der Vorrichtung unter etwa 3 mg liegt.

7. Überwachungsvorrichtung auf Enzymbasis nach einem der Ansprüche 1 bis 6, wobei der hermetisch abgedichtete Behälter aus einem oder mehreren feuchtigkeitsundurchlässigen Materialien besteht, die ausgewählt sind aus der Gruppe bestehend aus Glas, Siliziumdioxid, Metallen und Polymeren.

**8.** Verfahren zum Überwachen der Wärmeauswirkung einer Wärmeverarbeitung auf ein Objekt mit Hilfe einer Überwachungsvorrichtung auf Enzymbasis, wobei die Vorrichtung einen Behälter umfasst, der mindestens ein Enzym und mindestens eine Sperrschicht enthält, wobei das Verfahren die folgenden Schritte umfasst:

(a) Anordnen der Überwachungsvorrichtung auf Enzymbasis in Kontakt mit dem Objekt oder in der Nähe des Objekts;

(b) Aussetzen des Objekts und der Überwachungsvorrichtung auf Enzymbasis einer Wärmeverarbeitung bei einer Temperatur innerhalb eines Bereichs von 80°C bis 160°C über einen ausreichenden Zeitraum, um einen wesentlichen Teil des mindestens einen Enzyms abzubauen, ohne die mindestens eine Sperrschicht des Behälters aufzubrechen;

(c) Entfernen des Behälters aus dem Kontakt mit dem Objekt oder aus der Nähe des Objekts nach Vollendung von Schritt (b);

**dadurch gekennzeichnet, dass**:

- der Behälter ein hermetisch abgedichteter Behälter ist;
- der Behälter eine feste, dehydrierte Mischung umschließt, die das mindestens eine Enzym und mindestens ein erstes Füllmittel umfasst, wobei der Wassergehalt der dehydrierten Mischung unter 0,6 Gew.% ist, wobei die hermetische Abdichtung des hermetisch abgedichteten Behälters mit Hilfe der mindestens einen Sperrschicht erhalten wird, um einen Eintritt von Feuchtigkeit in den Behälter während der Wärmeverarbeitung des Objekts zu verhindern, und
- das Verfahren des Weiteren die folgenden Schritte umfasst:

(d) Öffnen des hermetisch abgedichteten Behälters und Erhalten einer Probe des mindestens einen Enzyms von dem hermetisch abgedichteten Behälter;

(e) Messen der Restaktivität des mindestens einen Enzyms in der erhaltenen Probe, und

(f) Verwenden der gemessenen Restaktivität als Mittel zum Quantifizieren der Wärmeauswirkunq der Wärmeverarbeitung von Schritt (b) auf ein oder mehrere gegebene Sollattribute des Objekts.

**9.** Verfahren zum Überwachen der Wärmeauswirkung einer Wärmeverarbeitung auf ein Objekt nach Anspruch 8, **dadurch gekennzeichnet, dass**:

- in Schritt (d) eine Probe des mindestens einen Enzyms, das in dem hermetisch abgedichteten Behälter eingeschlossen ist, in der Form einer Enzymlösung durch Löslichmachen der Fraktion der festen dehydrierten Mischung, die das mindestens eine Enzym umfasst, in einem oder mehreren Lösemitteln erhalten wird, und
- in Schritt (e) die Enzymlösung mit einem Substrat für das mindestens eine Enzym in Kontakt gebracht wird, was zu einem Produkt führt, und das Messen der Restaktivität des mindestens einen Enzyms durch Quantifizieren der Bildungsrate des Produkts erfolgt.

**10.** Verfahren zum Überwachen der Wärmeauswirkung einer Wärmeverarbeitung auf ein Objekt nach Anspruch 9, wobei das gegebene Sollattribut des Objekts, das in Schritt (f) quantifiziert wird, eine chemische, physikalische, organoleptische oder mikrobiologische Eigenschaft des Objekts ist.

**11.** Verfahren zum Überwachen der Wärmeauswirkung einer Wärmeverarbeitung auf ein Objekt nach einem der Ansprüche 8 bis 10, wobei das Objekt ausgewählt ist aus der Gruppe bestehend aus menschlicher oder tierischer Nahrung und medizinischen Werkzeugen oder Vorrichtungen.

**12.** Verfahren zum Überwachen der Wärmeauswirkung einer Wärmeverarbeitung auf ein Objekt nach einem der Ansprüche 8 bis 10, wobei das Objekt eine pharmazeutische Zusammensetzung in der Form einer Flüssigkeit, eines Sirups, einer Creme oder einer Paste ist.

**13.** Verfahren zum Überwachen der Wärmeauswirkung einer Wärmeverarbeitung auf ein Objekt nach einem der Ansprüche 8 bis 12, wobei das mindestens eine Enzym zwischen 0,001 und 10 Gew.% der festen dehydrierten Mischung darstellt.

**Revendications**

1. Dispositif de contrôle à base d'enzyme pour contrôler l'impact thermique d'un traitement thermique sur un objet à l'intérieur d'une plage de températures de 80 °C à 160 °C, ledit dispositif comprenant un conteneur contenant au moins une enzyme et au moins une barrière, dans lequel :

   - ledit conteneur est un conteneur hermétiquement scellé, et
   - ledit conteneur inclut un mélange déshydraté solide comprenant ladite au moins une enzyme et au moins un premier agent de remplissage, dans lequel la teneur en eau dudit mélange déshydraté est au-dessous de 0,6% en poids, le scellement hermétique du conteneur hermétiquement scellé étant obtenu au moyen de ladite au moins une barrière afin d'empêcher l'entrée d'humidité dans ledit conteneur.

2. Dispositif de contrôle à base d'enzyme selon la revendication 1, dans lequel ladite au moins une enzyme représente entre 0,001 et 10 % en poids du mélange déshydraté solide enfermé dans ledit conteneur hermétiquement scellé, et dans lequel ledit au moins un premier agent de remplissage représente entre 90 et 99,999 % en poids du mélange déshydraté solide enfermé dans ledit conteneur hermétiquement scellé.

3. Dispositif de contrôle à base d'enzyme selon la revendication 1 ou la revendication 2, dans lequel ledit au moins un premier agent de remplissage est un agent de remplissage non poreux.

4. Dispositif de contrôle à base d'enzyme selon l'une quelconque des revendications 1 à 3, dans lequel ledit au moins un premier agent de remplissage est un agent de remplissage inorganique ou un agent de remplissage organique.

5. Dispositif de contrôle à base d'enzyme selon l'une quelconque des revendications 1 à 4, dans lequel ledit mélange déshydraté solide comprend en outre au moins un second agent de remplissage qui représente jusqu'à 10 % en poids du mélange déshydraté solide enfermé dans ledit conteneur hermétiquement scellé.

6. Dispositif de contrôle à base d'enzyme selon l'une quelconque des revendications 1 à 5, dans lequel ladite au moins une enzyme est d'origine bactérienne, végétale, animale ou fongique et dans lequel la quantité de ladite au moins une enzyme dans ledit dispositif est au-dessous d'environ 3 mg.

7. Dispositif de contrôle à base d'enzyme selon l'une quelconque des revendications 1 à 6, dans lequel ledit conteneur hermétiquement scellé est fait d'une ou de plusieurs matières imperméables à l'humidité sélectionnées à partir du groupe constitué par le verre, la silice, des métaux et des polymères.

8. Procédé de contrôle de l'impact thermique d'un traitement thermique sur un objet au moyen d'un dispositif de contrôle à base d'enzyme, ledit dispositif comprenant un conteneur contenant au moins une enzyme et au moins une barrière, ledit procédé comprenant les étapes de :

   (a) placement dudit dispositif de contrôle à base d'enzyme en contact avec ledit objet ou au voisinage dudit objet ;
   (b) exposition dudit objet et dudit dispositif de contrôle à base d'enzyme à un traitement thermique à une température dans une plage de 80 °C à 160 °C pendant un temps suffisant pour dégrader une partie substantielle de ladite au moins une enzyme sans briser ladite au moins une barrière dudit conteneur,
   (c) suppression du contact dudit conteneur avec ledit objet ou du voisinage dudit objet après achèvement de l'étape (b) ;

   **caractérisé en ce que** :

   - ledit conteneur est un conteneur hermétiquement scellé,
   - ledit conteneur enferme un mélange déshydraté solide comprenant ladite au moins une enzyme et au moins un premier agent de remplissage, dans lequel la teneur en eau dudit mélange déshydraté est au-dessous de 0,6 % en poids, le scellement hermétique du conteneur hermétiquement scellé étant obtenu au moyen de ladite au moins une barrière pour empêcher l'entrée d'humidité dans ledit conteneur pendant un traitement thermique dudit objet, et
   - ledit procédé comprend en outre les étapes de :

      (d) ouverture dudit conteneur hermétiquement scellé et l'obtention d'un échantillon de ladite au moins une enzyme à partir dudit conteneur hermétiquement scellé,

(e) mesure de l'activité résiduelle de ladite au moins une enzyme dans l'échantillon obtenu, et

(f) utilisation de l'activité résiduelle mesurée en tant que moyen pour évaluer de manière quantitative l'impact thermique du traitement thermique de l'étape (b) sur un ou plusieurs attributs cibles donnés dudit objet.

9. Procédé de contrôle de l'impact thermique d'un traitement thermique sur un objet selon la revendication 8, **caractérisé en ce que** :

- dans l'étape (d), un échantillon de ladite au moins une enzyme enfermée dans ledit conteneur hermétiquement scellé est obtenu sous la forme d'une solution enzymatique en solubilisant dans un ou plusieurs solvants la fraction dudit mélange déshydraté solide comprenant ladite au moins une enzyme, et

- dans l'étape (e) ladite solution enzymatique est mise en contact avec un substrat pour ladite au moins une enzyme, aboutissant à un produit, et la mesure de l'activité résiduelle de ladite au moins une enzyme est effectuée en évaluant de manière quantitative la vitesse de formation dudit produit.

10. Procédé de contrôle de l'impact thermique d'un traitement thermique sur un objet selon la revendication 9, dans lequel ledit attribut cible donné dudit objet étant évalué de manière quantitative dans l'étape (f) est une propriété chimique, physique, organoleptique ou microbiologique dudit objet.

11. Procédé de contrôle de l'impact thermique d'un traitement thermique sur un objet selon l'une quelconque des revendications 8 à 10, dans lequel ledit objet est sélectionné à partir du groupe constitué par l'alimentation humaine ou animale et par des outils ou des dispositifs médicaux.

12. Procédé de contrôle de l'impact thermique d'un traitement thermique sur un objet selon l'une quelconque des revendications 8 à 10, dans lequel ledit objet est une composition pharmaceutique sous la forme d'un liquide, d'un sirop, d'une crème ou d'une pâte.

13. Procédé de contrôle de l'impact thermique d'un traitement thermique sur un objet selon l'une quelconque des revendications 8 à 12, dans lequel ladite au moins une enzyme représente entre 0,001 et 10 % en poids dudit mélange déshydraté solide.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5486459 A

### Non-patent literature cited in the description

- **Hendrickx et al.** *Critical reviews in Food Science and Nutrition,* 1995, vol. 35 (3), 231-262

- **Van Loey et al.** *Trends in Food Science & Technology,* 1996, vol. 7 (1), 16-26